# EUROPEAN PATENT SPECIFICATION

(11) **EP 0 506 884 B1**
(45) Date of publication and mention of the grant of the patent: **04.09.1996**
(21) Application number: 91903111.2
(22) Date of filing: 21.12.1990
(51) Int. Cl.: C12N 15/87, C12N 15/62, C12N 15/37, A61K 47/00, A61K 39/00

(54) **METHOD OF DELIVERING MOLECULES INTO EUKARYOTIC CELLS**
VERFAHREN ZUM TRANSPORT VON MOLEKÜLEN IN EUKARYOTENZELLEN
PROCEDE D'ACHEMINEMENT DE MOLECULES DANS DES CELLULES EUKARYOTES

(30) Priority: 21.12.1989 US 454450
(43) Date of publication of application: 07.10.1992
(73) Proprietor: WHITEHEAD INSTITUTE FOR BIOMEDICAL RESEARCH, Cambridge, MA 02142 (US); THE JOHNS HOPKINS UNIVERSITY SCHOOL OF MEDICINE, Baltimore, MD 21205 (US)
(72) Inventor: FRANKEL, Alan, Cambridge, MA 02139 (US); PABO, Carl, Newton, MA 02158 (US)
(74) Representative: VOSSIUS & PARTNER
(86) International application number: US9007607
(87) International publication number: WO9109958

(56) References cited:
- EP-A- 0 243 204
- EP-A- 0 388 758
- WO-A-88/05077
- CELL, vol. 55, 23 December 1988, Cell Press; A.D. FRANKEL et al., pp. 1189-1193
- CELLULAR & MOLECULAR BIOLOGY, vol. 28, no. 1, 1982, Pergamon Press Ltd., GB; J.C. STAVRIDIS et al., pp. 15-18
- PATENT ABSTRACTS OF JAPAN, vol. 12, no. 344 (C-528)(3191), 16 September 1988
- JOURNAL OF VIROLOGY, vol. 63, no. 1, 1989; pp. 1-8
- JOURNAL OF VIROLOGY, vol. 63, no. 3, 1989; pp. 1181-1187
- SCIENCE, vol. 240, 1988; pp. 70-73

## Description

### Background of the Invention

In eukaryotic cells, the nuclear envelope isolates the central genetic processes of DNA replication and RNA synthesis (i.e., transcription). The cell nucleus contains nucleic acids (i.e., DNA and RNA) and a variety of proteins. Some proteins are structural proteins that bind to and organize the DNA (e.g., histones). Other proteins are regulating proteins that bind to DNA and thereby positively or negatively regulate transcription (e.g., activator and repressor proteins). Still other proteins are enzymes that carry out DNA replication and RNA synthesis (e.g., DNA and RNA polymerase).

At the present time, there are several techniques available for introducing molecules, such as proteins and oligonucleotides, into cells. Such techniques are of interest, for example, in genetic engineering and as a possible means of introducing into cells drugs or other substances potentially of value diagnostically, therapeutically or prophylactically.

Presently-available genetic engineering transfer techniques include membrane fusion (e.g., fusion of cells with other cells or with liposomes), incubation of cells with a calcium phosphate precipitate of DNA fragment, DEAE-dextran-mediated transfection, electroporation, direct intracellular micro-injection of DNA fragments (e.g., via glass capillaries), and infection of cells with modified vectors (e.g., viral vectors).

All of these techniques can only be used in vitro to incorporate genetic material into cells in culture. In addition, the techniques are unreliable and nonspecific. Not all cells are altered and many cells do not survive the harsher treatments. Finally, these techniques are only used to transfer nucleic acids (e.g., DNA or RNA) into cells.

At the present time, there is no simple technique for delivering molecules of any type (e.g., proteins or peptides, nucleic acids) directly into the nucleus of cells in vitro or in vivo. It would be very useful if it were possible to deliver such molecules reliably and specifically into the cell nucleus.

### Summary of the Invention

The present invention pertains to the use of HIV Tat protein (Tat protein) to deliver a molecule of interest into eukaryotic cells, particularly into the cell nucleus, in vitro or in vivo. It further pertains to conjugates, which include a molecule of interest and HIV Tat protein, which are useful in the method of the present invention. The method of the present invention is based on the unexpected finding that when Tat protein is present extracellularly, it is readily taken up by cells and specifically introduced into the cell nucleus, as evidenced by the fact that cells treated with Tat exhibit high levels of transactivation.

In the method of the present invention, a molecule of interest and Tat protein are brought into contact with cells into which the molecule of interest is to be introduced, under conditions appropriate for its entry into cells. As a result, Tat protein and the molecule of interest enter into cells, in which they pass specifically into the nucleus.

In one embodiment of the present method, a molecule of interest-Tat protein conjugate, which includes a molecule of interest (i.e., a molecule to be introduced into cells) and Tat protein is brought into contact with cells into which the molecule of interest is to be introduced, under conditions appropriate for its entry into cells. As a result, the conjugate enters into cells, in which it passes specifically into the nucleus.

In a further embodiment of the present method, the molecule to be delivered into cells is a protein, a peptide or an oligonucleotide. The present method is particularly useful for delivery of proteins or peptides, such as regulatory factors, enzymes, antibodies, drugs or toxins, as well as DNA or RNA, into the cell nucleus.

A stabilizing agent, which serves to increase Tat stability and uptake, can be brought into contact with cells, in conjunction with the molecule of interest and Tat protein. For example, metal ions which bind to Tat protein and increase its stability and uptake, can be used for this purpose.

In a further embodiment, a lysosomotrophic agent is provided extracellularly in conjunction with Tat protein and a molecule of interest, in order to enhance uptake by cells. The lysosomotrophic agent can be used alone or in conjunction with a stabilizer. For example, lysosomotrophic agents such as chloroquine, monensin, amantadine and methylamine which have been shown to increase uptake of Tat in some cells by a few hundred fold, can be used for this purpose.

In another embodiment, a basic peptide, such as Tat 38-58 or protamine, is provided extracellularly with Tat and a molecule of interest to enhance uptake of Tat. Such basic peptides can also be used alone, in combination or with stabilizing agents or lysomotrophic agents.

Through use of the present method, it is possible to introduce into cells and, particularly into the cell nucleus, a drug or other substance which is of diagnostic, therapeutic or prophylactic value.

### Brief Description of the Drawings

Figure 1 is the amino acid sequence of the HIV-1 Tat protein.

Figure 2 is a thin layer chromatogram (TLC) showing chloramphenicol acetyl transferase (CAT) activity, a measure of Tat uptake, resulting from incubating HL3T1 cells with Tat for 24 hours at the indicated concentrations.

Figure 3 is a TLC showing CAT activity resulting from incubating HL3T1 cells with 5 µg of Tat and a variety of lysosomotrophic agents.

Figure 4 is a graph showing the cellular uptake and nuclear localization of ¹²⁵I-labeled Tat.

Figure 5 is a gel of nuclear fractions from HL3T1 cells treated with Tat protein in the absence (-) or presence (+) of chloroquine.

Figure 6 is a series of graphic representations showing chloroquine stimulation of Tat transactivation.

Figure 6A is a graph showing the effect of chloroquine concentration on uptake and transactivation by 2 µg Tat added to the medium.

Figure 6B is a graph showing the time course of uptake and transactivation by 2 µg of Tat and 100 µM chloroquine.

Figure 6C is a graph showing the effect on uptake and transactivation by several concentrations of Tat with 100 µM chloroquine.

Figure 7 is a TLC showing CAT activity from H9 lymphocytes, U937 promonocytes and HeLa cells treated with Tat protein in the absence or presence of chloroquine.

Figure 8 is a TLC showing CAT activity from HL3T1 cells and illustrating the extent of activation of the CAT reporter gene following various time periods of exposure to 1 µg Tat + 100 µM chloroquine.

Figure 9 is a schematic representation of the murine sarcoma virus (MSV) retroviral vector used to establish the H938 reporter cell line from H9 cells. The transcription start sites from the SV40 promoter, the HIV and MSV LTRs are indicated by arrows, and the location and size of the fragments protected in the RNase analysis are indicated by bars.

Figure 10 illustrates the results of an RNase protection experiment in H938 cells, using an α-³²P UTP-labeled HIV-1 LTR probe corresponding to a 200 bp fragment from -120 to +80 of the viral LTR, prepared by in vitro transcription.

Figure 11 is a graph illustrating the enhancement of transactivation in H938 cells upon addition of increasing amounts of the Tat 38-58 peptide with 1 µg of Tat as assayed by CAT activity after a 24 hour incubation with peptide and Tat.

Figure 12 is a graph illustrating transactivation of a tPA reporter gene under the control of the HIV-1 LTR in HeLa.318 cells upon addition of exongenous Tat or a TatE2C fusion protein.

### Detailed Description of the Invention

The present invention is based on the unexpected finding that Tat protein from immunodeficiency virus (e.g., HIV-1, HIV-2, SIV) is readily taken up into cells and subsequently into the cell nucleus. Tat is a potent viral transactivator and is essential for viral replication. In light of the fact that proteins and peptides are typically poorly taken up (Sternson, L.A., Ann. N.Y. Acad. Sci. 57:19-21 (1987)), the finding that Tat is readily taken up into cells is surprising.

As a result of this finding, it is now possible to use Tat protein to deliver molecules (e.g., proteins, peptides, nucleic acids) into cells and, specifically, into the cell nucleus. The present invention is a method of delivering a molecule of interest into cells and, particularly, of targeting a molecule to the cell nucleus, as well as a conjugate useful in the method. Any molecule can be delivered into cells, especially into the cell nucleus, using the method of the subject invention.

In one embodiment of the method of the present invention, a molecule of interest-Tat protein conjugate, which includes a molecule of interest (i.e., a molecule to be introduced into cells and delivered to the nucleus) attached to HIV Tat protein, is brought into contact with cells into which introduction of the molecule of interest is desired. The method can be used to deliver a molecule of interest either in vitro or in vivo. For example, delivery can be carried out in vitro by adding a molecule of interest-Tat conjugate to cultured cells, by producing cells that synthesize Tat or Tat conjugate or by combining a sample (e.g., blood, bone marrow) obtained from an individual with the conjugate, under appropriate conditions. Delivery can be carried out in vivo by administering the molecule of interest and Tat protein to an individual in whom it is to be used for diagnostic, preventative or therapeutic purposes.

The following is a description of: the HIV Tat protein; uptake of Tat into the cell nucleus; the molecule of interest-Tat protein conjugate; and the method by which the conjugate is used to deliver a selected substance into cells.

### HIV Tat protein

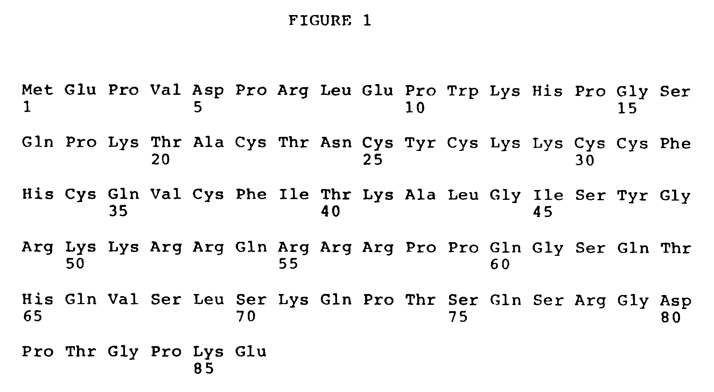

Figure 1 shows the amino acid sequence of the Tat protein. The full-length Tat protein is 86 amino acids long and is encoded by two exons; the N-terminal 72 residues are encoded by the first exon and the C-terminal 14 residues are encoded by the second exon. Tat protein contains a highly basic region (with 2 lysines and 6 arginines in 9 residues) and a cysteine-rich region (with 7 cysteines in 16 residues).

The basic region (i.e., amino acids 49-57) is thought to be important for nuclear localization. Ruben, S. et al., J. Virol. 63:1-8 (1989); Hauber, J. et al., J. Virol. 63:1181-1187 (1989). The cysteine-rich region mediates the formation of metal-linked dimers in vitro (Frankel, A.D. et al., Science 240:70-73 (1988)); (Frankel, A.D. et al., Proc. Natl. Acad. Sci, USA, 85:6297-6300 (1988)); and is essential for its activity as a transactivator (Garcia, J.A. et al., EMBO J. 7:3143 (1988); Sadaie, M.R. et al., J. Virol. 63:1 (1989)). Like other regulatory proteins, the N-terminal region may be involved in protection against intracellular proteases (Bachmair, A. and A. Varshavsky, Cell, 56:1019-1032 (1989)).

It will be appreciated that the entire 86 amino acids which make up the Tat protein may not be required for the uptake activity of Tat. For example, a protein fragment or a peptide which has fewer than the 86 amino acids, but which exhibits uptake into cells and uptake into the cell nucleus, can be used (a functionally effective fragment or portion of Tat). As is shown in the Examples below, Tat protein containing residues 1-72 is sufficient for uptake activity and Tat residues 1-67 are shown to mediate the entry of a heterologous protein into cells. In addition, a synthetic peptide containing Tat residues 1-58 has now been shown to have uptake activity. A Tat peptide comprising the region that mediates entry and uptake into cells can be further defined using known techniques. (see, e.g., Frankel, A.D., et al., Proc. Natl. Acad. Sci, USA, 86: 7397-7401 (1989)).

The Tat peptide can be a single (i.e., continuous) amino acid sequence present in Tat protein or it can be two or more amino acid sequences which are present in Tat protein, but in the naturally-occurring protein are separated by other amino acid sequences. As used herein, Tat protein includes a naturally-occurring amino acid sequence which is the same as that of naturally-occurring Tat protein, its functional equivalent or functionally equivalent fragments thereof (peptides). Such functional equivalents or functionally equivalent fragments possess uptake activity into the cell and into the cell nucleus that is substantially similar to that of naturally-occurring Tat protein. Tat protein can be obtained from naturally-occurring sources or can be produced using genetic engineering techniques or chemical synthesis.

The amino acid sequence of naturally-occurring HIV Tat protein can be modified, by addition, deletion and/or substitution of at least one amino acid present in the naturally-occurring Tat protein, to produce modified Tat protein (also referred to herein as Tat protein). Modified Tat protein or Tat peptide analogs with increased stability can thus be produced using known techniques. Therefore, Tat proteins or peptides may have amino acid sequences which are substantially similar, although not identical, to that of naturally-occurring Tat protein or portions thereof. In addition, cholesterol or other lipid derivatives can be added to Tat protein to produce a modified Tat having increased membrane solubility.

Variants of Tat protein can be designed to modulate the intracellular location of Tat and the molecule of interest following uptake into the cell or when expressed in the cell. When added exogenously, such variants are designed such that the ability of Tat to enter cells is retained (i.e., the uptake of the variant Tat protein or peptide into the cell is substantially similar to that of naturally-occurring HIV Tat). For example, alteration of the basic region thought to be important for nuclear localization (see e.g., Dang, C.V. and Lee, W.M.F., J. Biol. Chem. 264: 18019-18023 (1989); Hauber, J. et al., J.Virol. 63: 1181-1187 (1989); Ruben, S.A. et al., J. Virol. 63: 1-8 (1989)) can result in a cytoplasmic location or partially cytoplasmic location of Tat, and therefore, of the molecule of interest. Alternatively, a sequence for binding a cytoplasmic component can be introduced into Tat in order to retain Tat and the molecule of interest in the cytoplasm or to confer regulation upon nuclear uptake of Tat and the molecule of interest.

### Demonstration of Uptake of Tat into the Cell Nucleus

In an attempt to develop a convenient assay for the Tat protein, various ways of introducing Tat protein into cells containing a reporter gene (i.e., a gene that can be transcribed to express an assayable protein, e.g., the chloroamphenicol acetyl-transferase or CAT gene) were tried. Scrape loading, as described by McNeil and co-workers, was shown to be a convenient method which gave quantitative and reproducible transactivation of the HIV-1 promoter (McNeil, P.L. et al., J. Cell Biol., 98:1556-1564 (1984)). It is believed that scrape-loading transiently damages the cell membrane and allows molecules present in the culture medium to equilibrate with the cytoplasm. An unexpected result was seen when Tat was simply added to the culture medium of HL3T1 cells (HeLa cells containing the integrated LTR-CAT plasmid): Expression of CAT from the integrated HIV-1 promoter increased and was proportional to the Tat concentration, indicating that Tat was taken up and transactivated the HIV-1 promoter. This result was surprising because proteins and peptides are generally believed to be poorly taken up by cells. Sternson, L.A., Ann. N.Y. Acad. Sci., 57, 19-21 (1987).

To measure cellular uptake directly, HL3T1 cells were treated with ¹²⁵I-labeled Tat in the presence or absence of chloroquine, and the amount of radioactive Tat present in various cellular fractions was determined. This work is described in greater detail in Example III.

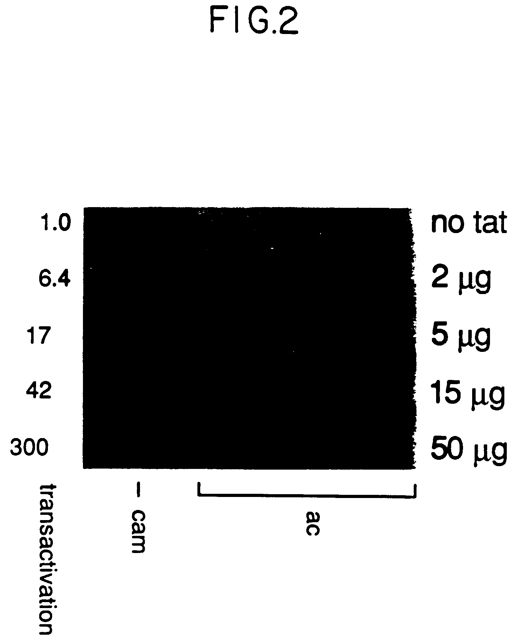

Figure 2 shows results of assessment of the expression of CAT by HL3T1 cells incubated with Tat protein for 24 hours, at the indicated concentrations. Expression of CAT from the integrated HIV-1 promoter increased and was proportional to the Tat concentration. CAT activity did not increase further after 24 hours. Additional small increases in activity (2- to 3-fold) were observed upon addition of 10 mM zinc or 1 mM cadmium, suggesting that metals might stabilize Tat either during uptake or once inside the cell.

To explore the uptake process further, various lysosomotrophic agents were added to the culture medium. Lysosomotrophic agents are thought to inhibit receptor-mediated endocytosis. Mellman, I. et al., Ann. Rev. Biochem., 55:663-700 (1986).

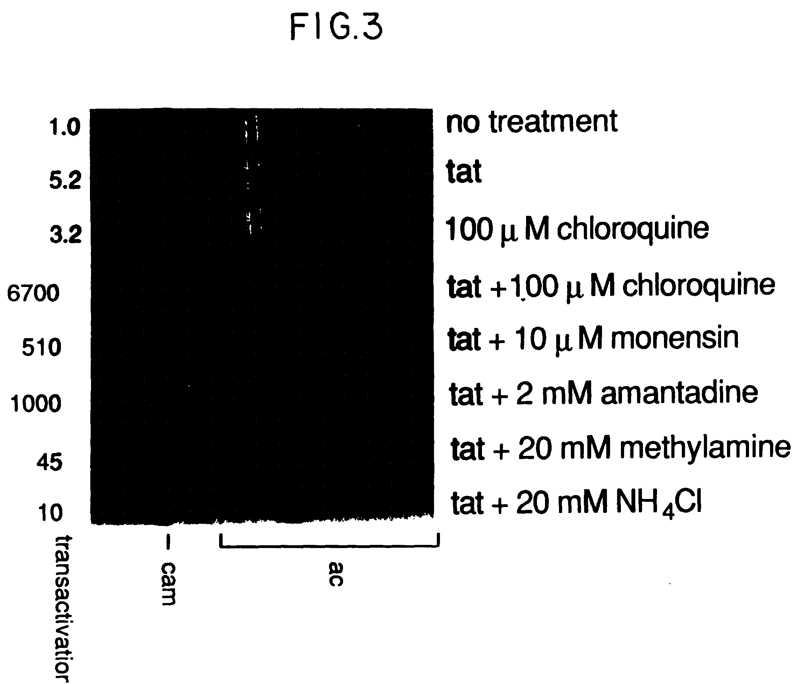

Figure 3 shows the effect that a variety of lysosomotrophic agents have on uptake and subsequent transactivation by Tat placed in tissue culture medium. HL3T1 cells were incubated with 5 µg of Tat (100 nM) and each agent for 24 hours, the medium was replaced, and CAT activity was determined after 60 hours. Activity from untreated cells, cells incubated with Tat alone and cells incubated with chloroquine alone are also shown. The level of uptake and subsequent transactivation in HL3T1 cells by 5 µg of Tat with chloroquine present was about 7000-fold compared with untreated cells, whereas chloroquine gave little increase in promoter activity in the absence of Tat. Monensin, amantadine and methylamine also significantly increased transactivation, whereas ammonium chloride only slightly increased activity. No lysosomotrophic agent tested significantly activated the promoter in the absence of Tat. The parameters of chloroquine-stimulated Tat activity are explained in more detail in Example IV.

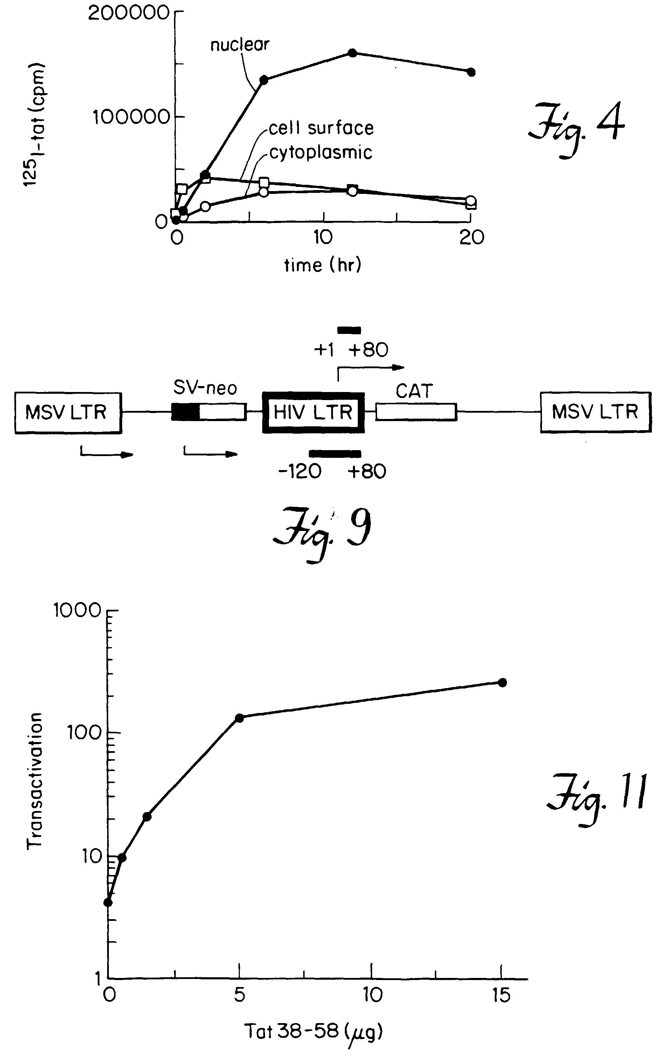

Figure 4 shows that within 6 hours after treating HL3T1 cells with Tat and chloroquine, a significant amount of radioactive Tat (about 3% of the total) had been taken up by the cells. Most of this Tat (<80%) was localized in the nuclear fraction. Trypsin-sensitive counts, representing Tat bound to the cell surface, remained relatively constant and by 12 hours were less than 20% of the counts found in the nucleus.

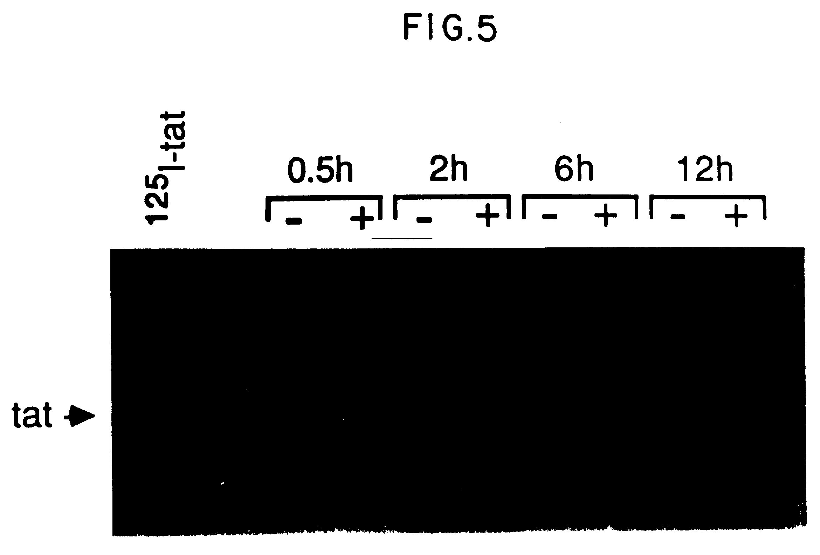

Figure 5 shows nuclear extracts run on an SDS gel. A radioactive band comigrating with intact Tat is readily apparent. HL3T1 cells treated with Tat but without chloroquine showed similar kinetics of uptake and nuclear localization when assayed by counting the cellular fractions but only degraded Tat was seen on the gel.

The ability of Tat to directly enter lymphocytes or monocytes was also assessed; Tat readily entered both types of cells, as demonstrated by the high levels of transactivation in cells treated with Tat, alone or with chloroquine. H9 lymphocytes and U937 promonocytes (10⁶ cells) containing an integrated HIV-1 LTR-CAT plasmid (H938 and U38 cells, respectively (Felber, B.K. and G.N. Pavlakis, Science, 239:184 (1988)) were incubated in RPMI 1640 medium containing 10% fetal bovine serum (1 ml in 25 mm wells) at 37°C (no tat), treated with 5 µg of Tat protein (tat) or treated with 5 µg of Tat and 100 µm chloroquine (tat + CQ). Cells were harvested 24 hours after Tat treatment and assayed for CAT activity (Gorman, C.M. et al., Mol. Cell Biol., 2:1044 (1982). HeLa cells (10⁶ cells) containing an integrated HIV-1 LTR-CAT plasmid (HL3T1) (Felber, B.K. and G.N. Pavlakis, Science, 239:184 (1988)), were incubated in Dulbecco's modified Eagle's medium (DMEM) with 10% fetal bovine serum (1 ml in 25 mm wells) and similarly treated with Tat protein, or with Tat and chloroquine, and assayed for CAT activity. Unacetylated (cm) and acetylated (ac) forms of ¹⁴C chloramphenicol were separated by thin layer chromatography.

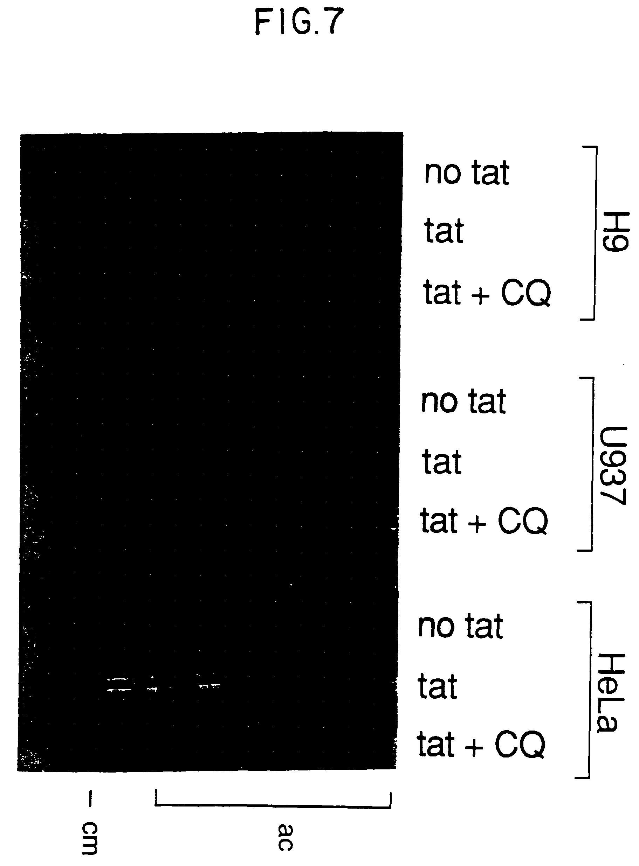

Figure 7 shows the results of this assessment of Tat entry into lymphocytes and monocytes. High levels of transactivation were seen in all three cell lines. In the HeLa cells, the addition of chloroquine resulted in a significant stimulation of Tat activity. However, in contrast to the case with HeLa cells, chloroquine had little effect on Tat entry into lymphocytes or monocytes. The chloroquine-independent entry into lymphocytes and monocytes may suggest a different mechanism of uptake.

The time course of binding was determined in HeLa cells containing an integrated HIV-1 LTR-CAT plasmid (HL3T1 cells) (Felber, B.K. and G.N. Pavlakis, Science, 239:184 (1988)). Cells (2 X 10⁶) were grown to confluence in 12 well tissue culture plates (12mm well diameter), and washed with phosphate buffered saline (PBS). Cells were incubated in fresh DMEM with 1 µg Tat (1-72) and 100 µM chloroquine at 37°C for different lengths of time. Following two washes with PBS to remove Tat, fresh medium was added and transactivation was measured 24 hours after Tat addition. CAT activity was used as a measure of transactivation.

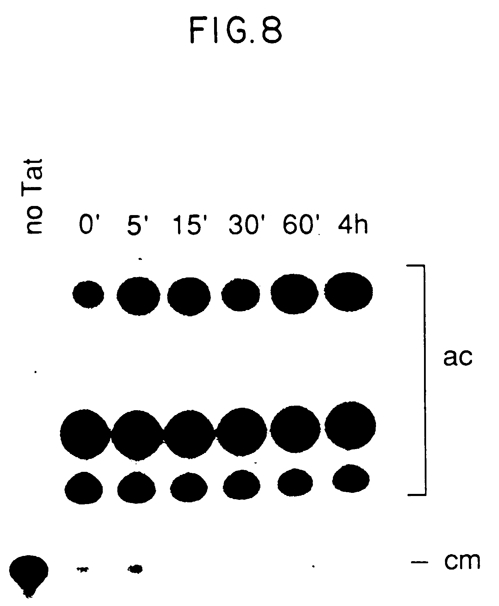

The results of this analysis are shown in Figure 8. The basal level of expression from the HIV-1 LTR in the absence of Tat is shown in the "no Tat" lane. Maximal levels of transactivation were observed after a five minute exposure to Tat. Thus, binding is rapid, and a brief exposure can result in uptake by cells, as assayed by transactivation.

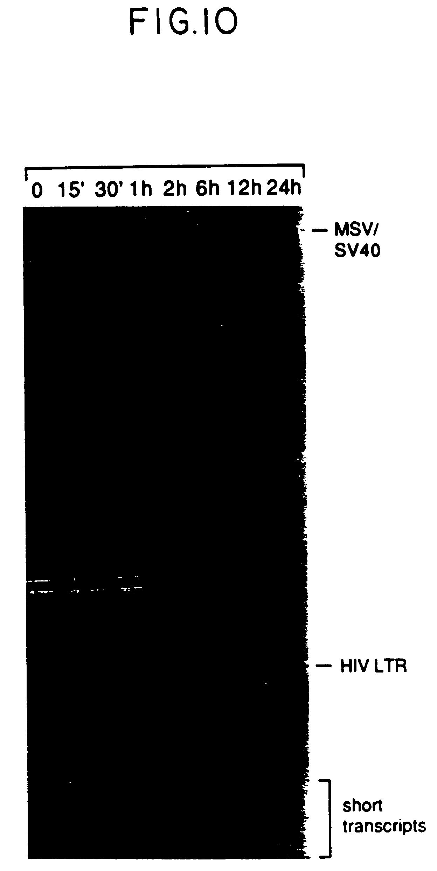

The time required to observe a response to exogenous Tat was determined in H938 cells. H938 cells were derived from the H9 lymphoid cell line by infection with a murine sarcoma virus (MSV) retroviral vector. (Felber, B.K. and G.N. Pavlakis, Science, 239:184 (1988)). The integrated MSV vector contains the CAT gene under the control of the HIV-1 LTR, and the neo gene under the control of an SV40 promoter (Figure 9). H938 cells were maintained in RPMI 1640 medium supplemented with 10% fetal bovine serum, penicillin (250 U/ml), and streptomycin (250 µg/ml). The cells were treated with 10 µg/ml of Tat protein (amino acids 1-72) in the presence of 100 µg/ml protamine, and RNA was prepared and analyzed by RNase protection. An α-³²P UTP-labeled HIV-1 LTR probe corresponding to a 200 bp fragment from -120 to +80 of the viral LTR was prepared by in vitro transcription. These procedures are further described in Example V.

The results of the RNase protection assay are shown in Figure 10. Two major fragments were protected. The 80 nucleotide fragment is derived from transcripts expressed from the HIV LTR and the 200 nucleotide fragment is derived from transcripts expressed from either the upstream MSV or SV40 promoters. Transcription from the HIV LTR increased after 15 minutes of exposure to Tat, and reached a maximum by 2-6 hours. In contrast, transcription from the upstream MSV and SV40 promoters was not increased by Tat addition, indicating that exogenously added Tat retains specificity for the HIV promoter. When Tat protein is added exogenously to cells, there is a significant increase in transcription in 15 minutes, indicating that Tat can enter cells, become localized to the nucleus, bind to its target site TAR specifically, and promote transcription within 15 minutes. (The short transcripts, which may be degradation products from incompletely elongated RNAs, were not affected by Tat.)

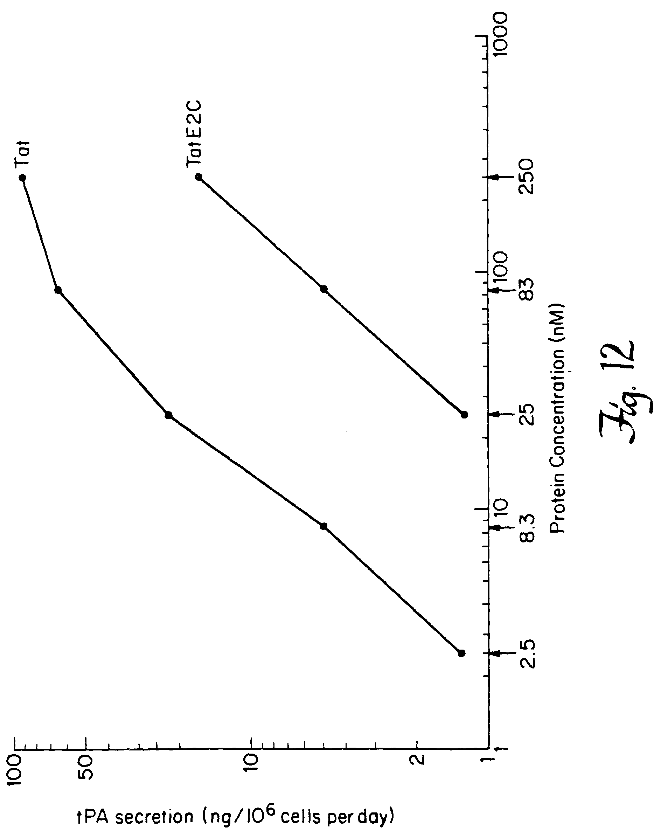

Several peptide fragments of Tat were tested for their ability to compete for Tat binding and uptake in HL3T1 and H938 cells. In these experiments, 0.5 X 10⁶ H938 cells were pelleted and resuspended in 0.5 ml fresh RPMI 1640 medium. Cells were incubated at 37°C with 1 µg Tat (1-72) and increasing concentrations of peptide. Extracts were prepared after 24 hours and assayed for CAT activity. Surprisingly, Tat 38-58, which contains the basic region of Tat, actually enhanced the effect of exogenous Tat and increased transactivation in a concentration dependent manner. Figure 11 shows the results of this experiment in the H938 cell line. The data was quantitated by cutting the spots from the TLC plates and counting the associated radioactivity in a scintillation counter.

Protamine (protamine sulfate, Sigma), another basic peptide, was also observed to enhance transactivation by extracellular Tat when present at a concentration of 100 µg/ml. However, a smaller Tat peptide, containing only the basic region from 47-58, had no effect on transactivation under the conditions used. A mixture of two peptides, Tat 38-47 and Tat 48-58, the products of chymotryptic digestion of Tat 38-58, also had no effect on transactivation under these conditions. No enhancement of activity by protamine was seen when HL3T1 cells were scrape-loaded with Tat, suggesting that protamine directly affects the uptake process.

Other cell lines were also tested for Tat uptake and transactivation activity. Jurkat T cells showed significant transactivation when Tat was added to the medium and showed further transactivation in the presence of chloroquine. A Vero line (VNHIV-CAT; Mosca, J.D. et al., Nature, 325:67-70 (1987)) also showed significant transactivation upon incubation of cells with Tat and chloroquine; no activity was seen with Tat alone. However, since the basal expression of CAT was low in this cell line, a several fold increase in CAT activity would still have been undetectable.

To directly follow the entry of Tat into live cells, Tat was labelled with rhodamine (TRITC-Tat) and its movement was followed by fluorescence microscopy. Punctate staining was observed on the surface of HL3T1 and H938 cells immediately after incubation with TRITC-Tat, similar to that seen in receptor-mediated endocytosis. After one hour, clear nuclear staining was observed in HL3T1 cells. Punctate cytoplasmic staining was also observed, suggesting that Tat may be localized within endosomes. Incubation at low temperature, which blocks endocytosis, also blocked entry of rhodamine-labeled Tat. After six hours, most of the Tat was in the nucleus of HL3T1 cells, but was excluded from the nucleoli. Remarkably, every cell in the culture was labeled with TRITC-Tat, indicating that the uptake of exogenous Tat is efficient. (Cellular localization was also examined in H938 cells, however, since the nucleus constitutes most of the lymphocytic cell, it was difficult to distinguish nuclear from non-nuclear compartments.) When tested for transactivation, TRITC-Tat was found to have the same specific activity as unmodified Tat.

### Tat-mediated uptake of a heterologous protein

A preliminary assessment of the ability of Tat to mediate the uptake of a molecule of interest was carried out. Additional details of this analysis are provided in Example VII. The E2 open reading frame of the bovine papillomavirus-1 (BPV-1; Chen, E.Y. et al., Nature 299: 529-534 (1982)) encodes both positive and negative acting transcriptional regulators (regulatory factors; Sousa, R. et al., Biochim. Biophys. Acta 1032: 19-37 (1990); Lambert, P.F et al., J. Virol. 63(7): 3151-3154 (1989); Lambert, P.F. et al., Cell 50: 69-78 (1987)). A fusion gene was constructed in which the HIV-1 tat gene was linked to the carboxy-terminal region of the E2 open reading frame. The construct which encodes the fusion protein, pFTE103 (constructed by Dr. J. Barsoum, Biogen, Inc.), was designed to express a protein comprising amino acids 1 through 67 of Tat at the amino terminus, followed by the C-terminal 105 amino acids of E2 (residues 306 through 410 of BPV-1 E2), which contain the DNA binding domain of the E2 open reading frame (EP 0,302,758, Androphy et al., (Feb. 6, 1989); Giri, I. and Yaniv, M., EMBO J., 7(9): 2823-2829 (1988); McBride, A.A. et al., EMBO J. 7(2): 533-539 (1988); Androphy, E.J. et al., Nature 325: 70-73 (1987)). pFTE103 was introduced into E. coli and the TatE2C fusion protein was expressed using the T7 RNA polymerase expression system as described by Studier et al. (Studier et al., Methods in Enzymology 185:60-89 (1990)). The purified TatE2C fusion protein migrated with an apparent molecular weight of 20,000 to 21,000 daltons on protein gels. Uptake of the TatE2C fusion protein was tested following introduction into the culture medium of animal cells.

Uptake of the Tat portion of the fusion protein (molecule of interest-Tat protein conjugate) was assayed by measuring transactivation of a Tat-responsive reporter construct integrated into HeLa cells (HeLa.318 cells). The Tat-responsive reporter construct (pXB318) present in HeLa.318 cells contains the human tissue plasminogen activator (tPA) cDNA reporter gene from pTPA114 (Fisher et al. J. Biol. Chem. 260:11223-11230 (1985)) under the control of the HIV-1 long terminal repeat (LTR) from pU3R-III (Sodroski et al. Science 227:171-173 (1985)). Tat protein (amino acids 1-72) or the TatE2C fusion protein were added to the culture medium of HeLa.318 cells in 24 well plates at concentrations ranging from 2.5 nM to 250 nM, in the presence of 100 µM chloroquine, essentially as described (Frankel, A.D. and Pabo, C.O., Cell 55:1189-1193 (1988)). The culture medium was harvested 24 hours later and assayed for tPA activity by the method of Granelli-Piperno and Reich (J. Exp. Med. 148:223-234 (1978)). Cell numbers were determined and tPA secretion was expressed as ng/10⁶ cells per day. Figure 12 shows the results obtained from a tPA assay of HeLa.318 media 24 hours after the addition of Tat or TatE2C protein to culture medium. In the absence of Tat or the TatE2C protein, tPA activity was undetectable (less than 0.1 ng/10⁶ cells per day). However, addition of either Tat or TatE2C protein led to an increase in tPA production (Figure 12). Thus, it appears that Tat (residues 1-67) can retain the ability to enter cells when linked to a heterologous protein.

Although transactivation upon addition of the TatE2C protein was somewhat less efficient than that observed upon addition of Tat, the TatE2C fusion protein was also less active than Tat in transactivation assays when the proteins were produced intracellularly after transfection of the genes into HeLa.318 cells. Thus, it is not clear whether the apparent reduction in activity is attributable to reduced uptake or reduced activity of the fusion protein produced by E. coli and added exogenously. It is possible that some Tat activity may be lost during the denaturation and refolding of the TatE2C fusion protein during purification.

Uptake of the E2 portion of the conjugate was determined by indirect immunofluorescence using rabbit polyclonal serum raised against E2-C85 (the C-terminal 85 amino acids of the E2 protein produced in E. coli). For indirect immunofluorescence, mouse 3T3 cells were seeded into LAB-TEK four chamber tissue culture chamber/slides. The next day, TatE2C fusion protein was added at 250 nM to the culture medium, in the presence of 100 µM chloroquine. Six hours later; immunofluorescence was performed as described in Example VII.

While only very faint background fluorescence was seen when E2.C85 protein was added to cells (at the same concentration and in the presence of 100 µM chloroquine), addition of the TatE2C fusion protein led to very intense fluorescence in all cells observed. These cells displayed fluorescence on the plasma membrane, in the cytosol and in nuclei. The staining was present in bright patches rather than evenly dispersed throughout the cells. The amount of E2 fluorescence obtained following addition of TatE2C protein to culture medium was far greater than the immunofluorescence observed when a TatE2C gene was expressed in these same cells. These data indicate that the Tat protein is capable of efficiently carrying a heterologous protein present as part of a molecule of interest-Tat conjugate into cells.

### The molecule of interest-Tat protein conjugate

A molecule of interest, which will generally be a protein or peptide, a nucleotide sequence, or other chemical which has diagnostic, prophylactic or therapeutic application (referred to herein as a drug) is combined, as described below, with HIV Tat protein to produce a molecule of interest-Tat protein conjugate and the resulting conjugate is brought into contact with the extracellular surface of cells.

In one embodiment of the present invention, the molecule of interest is a protein, such as an enzyme, antibody, toxin, or regulatory factor (e.g., transcription factor) whose delivery into cells, and particularly into the cell nucleus is desired. For example, some viral oncogenes inappropriately turn on expression of cellular genes by binding to their promoters. By providing a competing binding protein in the cell nucleus, viral oncogene-activity can be inhibited.

In a further embodiment, the molecule of interest is a nucleotide sequence to be used as a diagnostic tool (or probe), or as a therapeutic agent, such as an oligonucleotide sequence which is complementary to a target cellular gene or gene region and capable of inhibiting activity of the cellular gene or gene region by hybridizing with it. In yet another embodiment, the molecule of interest is a drug, such as a peptide analog or small molecule enzyme inhibitor, whose introduction specifically and reliably into the cell nucleus is desired.

The molecule of interest can be obtained or produced using known techniques, such as chemical synthesis, genetic engineering methods and isolation from sources in which it occurs naturally. The molecule of interest can be combined with or attached to the Tat protein to form the molecule of interest-Tat protein conjugate which is a subject of the present invention.

The attachment of the molecule of interest to Tat to produce a molecule of interest-Tat protein conjugate may be effected by any means which produces a link between the two constituents which is sufficiently stable to withstand the conditions used and which does not alter the function of either constituent. Preferably, the link between them is covalent. For example, recombinant techniques can be used to covalently attach Tat protein to molecules, such as by joining the gene coding for the molecule of interest with the gene coding for Tat and introducing the resulting gene construct into a cell capable of expressing the conjugate. Alternatively, the two separate nucleotide sequences can be expressed in a cell or can be synthesized chemically and subsequently joined, using known techniques. Alternatively, the protein of interest-Tat molecule can be synthesized chemically as a single amino acid sequence (i.e., one in which both constituents are present) and, thus, joining is not needed.

Coupling of the two constituents can be accomplished via a coupling or conjugating agent. There are several intermolecular cross-linking reagents which can be utilized (see, for example, Means, G.E. and Feeney, R.E., Chemical Modification of Proteins, Holden-Day, 1974, pp. 39-43). Among these reagents are, for example, J-succinimidyl 3-(2-pyridyldithio) propionate (SPDP) or N, N'-(1,3-phenylene) bismaleimide (both of which are highly specific for sulfhydryl groups and form irreversible linkages); N, N'-ethylene-bis-(iodoacetamide) or other such reagent having 6 to 11 carbon methylene bridges (which is relatively specific for sulfhydryl groups); and 1,5-difluoro-2,4-dinitrobenzene (which forms irreversible linkages with amino and tyrosine groups). Other cross-linking reagents useful for this purpose include: p,p'-difluoro-m, m'-dinitrodiphenylsulfone (which forms irreversible cross-linkages with amino and phenolic groups); dimethyl adipimidate (which is specific for amino groups); phenol-1,4-disulfonylchloride (which reacts principally with amino groups); hexamethylenediisocyanate or diisothiocyanate, or azophenyl-p-diisocyanate (which reacts principally with amino groups); glutaraldehyde (which reacts with several different side chains) and bisdiazobenzidine (which reacts primarily with tyrosine and histidine).

### Delivery of a molecule of interest using the present method

The present method can be used to deliver a molecule of interest into cells, particularly into the cell nucleus, in vitro or in vivo. In in vitro applications in which the molecule is to be delivered into cells in culture, the molecule of interest-Tat protein conjugate is simply added to the culture medium. This is useful, for example, as a means of delivering into the nucleus substances whose effect on cell function is to be assessed. For example, the activity of purified transcription factors can be measured, or the in vitro assay can be used to provide an important test of a molecule's activity, prior to its use in in vivo treatment.

Alternatively, the molecule of interest-Tat protein conjugate can be used for prophylactic or therapeutic purposes (for the treatment, prophylaxis or diagnosis of a disease or condition). For example, the molecule of interest-Tat protein conjugate can be combined with a sample obtained from an individual (e.g., blood, bone marrow) in order to introduce the molecule of interest into cells present in the sample and, after treatment in this manner, the sample returned to the individual. A series of treatments carried out in this manner can be used to prevent or inhibit the effects of an infectious agent. For example, blood can be removed from an individual infected with HIV or other viruses, or from an individual with a genetic defect. The blood can then be combined with a molecule of interest-Tat protein conjugate in which the molecule of interest is a drug capable of inactivating the virus or an oligonucleotide sequence capable of hybridizing to a selected virus sequence and inactivating it or a protein that supplements a missing or defective protein, under conditions appropriate for entry into cells of the conjugate and maintenance of the sample in such a condition that it can be returned to the individual. After treatment, the blood is returned to the individual.

Alternatively, a molecule of interest-Tat protein conjugate can be delivered in vivo. For example, cells that synthesize Tat conjugate can be produced and implanted into an individual so that Tat conjugate is constantly present. In another embodiment, the conjugate can be used much like a conventional therapeutic agent and can be a component of a pharmaceutical composition which includes other components useful, for example, for delivery, stability or activity of the conjugate. In this embodiment, a molecule of interest-Tat protein conjugate, such as a selected oligonucleotide sequence-Tat protein conjugate, can be administered in sufficient quantity to result in entry into cells, particularly cell nuclei, and inhibition (reduction or elimination) of the causative agent (e.g., virus or bacterium) or provision of a missing or defective protein. Administration of a molecule of interest-Tat protein conjugate may be by a variety of routes. For example, administration may be by injection, infusion or other parenteral routes (e.g., subcutaneous, intraveous, intramuscular, intrasternal and intracranial injection or infusion techniques). Similarly, administration may be topical (administered topically), that is, applied locally to a particular part of the body (e.g., skin, lower intestinal tract, vaginally, rectally) where appropriate. For example, in the case of a papillomavirus infection, topical administration would be an appropriate mode of administration.

A molecule of interest-Tat protein conjugate can also be used in making a vaccine. For example, the molecule of interest can be an antigen from the bacteria or virus or other infectious agent that the vaccine is to immunize against (e.g., pg120 of HIV). Providing the antigen into the cell cytoplasm allows the cell to process the molecule and express it on the cell surface. Expression of the antigen on the cell surface will raise a killer T-lymphocyte response, thereby inducing immunity.

For example, for in vivo applications, a selected conjugate can be formulated in appropriate compositions which include pharmacologically appropriate carriers, adjuvants and vehicles. In general, these carriers include aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. In addition, intravenous vehicles can include fluid and nutrient replenishers, and electrolyte replenishers, such as those based on Ringer's dextrose. Preservatives and other additives can also be present, such as, for example, antimicrobials, antioxidants, chelating agents, and inert gases. See, generally, Remington's Pharmaceutical Sciences, 16th Ed., Mack, ed. 1980. The amount of conjugate administered will vary and will depend on such factors as the condition or disease in question, the mode of administration, and the individual's size.

### Examples

The subject invention will now be illustrated by the following examples, which are not to be seen as limiting in any way.

### Example I Bacterial Expression and Purification of Tat

Two plasmids were constructed to produce the Tat protein in E. coli; one expresses amino acids 1-86 (the entire coding sequence) and the other expresses the first coding exon of Tat (residues 1-72). It is known that the second exon is not required for activity (Cullen, B.R., Cell, 46: 973-982 (1986); Muesing, M.A., et al., Cell, 48: 691-701 (1987); Sodroski, J., et al., Science, 229: 74-77 1985); Frankel, A.D., et al., Proc. Natl. Acad. Sci., USA, 86:7397-7401 (1989)). Synthetic tat genes were constructed and ligated into the NdeI site of pET-3a, a plasmid that uses a strong bacteriophage T7 promoter to express cloned genes (Studier, F.W. and B.M. Moffat, J. Mol. Biol., 189: 113-130 (1986); Rosenberg, A.H., et al., Gene, 56: 125-135 (1987)). The resulting plasmids, ptat72 and ptat86, express Tat (residues 1-72 or 1-86, respectively) as 1%-5% of total E. coli protein. Both proteins gave similar results in all experiments. BL21(DE3) cells were used for expression and these cells also contained a plasmid expressing the T7 lysozyme gene to inhibit any T7 RNA polymerase expressed prior to induction (F.W. Studier, personal communication). Tat was induced with isopropyl β-D-thiogalactopyranoside (IPTG) (Studier, F.W. and B.M. Moffat, J. Mol. Biol., 189: 113-130 (1986)) and purified essentially as described (Frankel, A.D., et al., Science, 240: 70-73 (1988)) except that Tat was extracted from the polyethyleneimine pellet with 10% ammonium sulfate instead of 700 mM KCl, and the S-Sepharose chromotography was eliminated.

### Example II Synthetic Tat Peptides

Syntheses were performed using Fmoc chemistry on a Milligen/Biosearch model 9600 peptide synthesizer with a peptide amide linker-norleucine-4-methylbenzhydrylamine (PAL-Nle-MBHA) polystyrene resin (Milligen/Biosearch; 0.5 g). The benzotriazolyloxytris(dimethylamino)phosphonium hexafluorophosphate/1-hydroxybenzotriazole (BOP/HOBt) coupling method (Hudson, D., J. Org. Chem., 53: 617-624 (1988)) was used with coupling times of 1-4 hours and with double coupling of His-33. Protecting groups were t-butyl ester (for Glu and Asp), 2,2,5,7,8-pentamethylchroman-6-sulfonyl (Arg), t-butyloxycarbonyl (Lys), trityl (His and Cys), t-butyl (Ser, Thr, and Tyr), and trimethoxybenzyl (Asn and Gln). All peptides were synthesized as their C-terminal amides. After synthesis was completed, protecting groups were removed and the peptide chains were cleaved from the resin with trifluoroacetic acid/ethanedithiol/thioanisole/anisole (90:3:5:2, vol/vol). The mixture was filtered and the products were obtained by addition of cold anhydrous diethyl ether to the filtrate. The precipitate was collected by filtration, thoroughly washed with ether and dried.

Peptides were treated with 0.5 M dithiothreitol at 37°C for 30 minutes to ensure complete reduction of the cysteines and were purified on a C₄ HPLC column (Vydac) using an acetonitrile gradient in 0.1% trifluoroacetic acid. Amino acid composition was determined by hydrolysis in 6 M HCl containing 0.5% phenol at 100°C and analysed on a LKB Alpha Plus analyzer. Peptide purity (>90%) was determined by HPLC using an acetonitrile gradient of <0.5% per minute.

### Example III Uptake of ¹²⁵-I-Labeled Tat

Tat (residues 1-72) was labeled with ¹²⁵I by treating 500 µg of protein with 0.5 mCi ¹²⁵I and IODO-BEADS (Pierce) in 0.1 M Tris-HCℓ (pH 7.5) at room temperature for 5 minutes. The sample was dialyzed to remove unreacted ¹²⁵I. The specific activity was approximately 10⁶ cpm/µg protein. HL3T1 cells (2 x 10⁶ cells per dish) were treated with 5 µg radioactive Tat in the presence or absence of 100 µM chloroquine. Medium was removed at various times, cells were washed with PBS and EDTA, and cells were trypsinized for 10 minutes. Pancreatic trypsin inhibitor was added (5 µg/ml), cells were chilled to 4°C, centrifuged at 100Xg, and the supernatant was saved. The cell pellet was washed twice with serum-free DMEM, once with PBS and nuclei were isolated by lysis in 0.5% NP-40 as described (Ausubel, F.M. et al., Current Protocols in Molecular Biology (New York: John Wiley and Sons, 1987)). ¹²⁵I was counted using an LKB gamma counter.

### Example IV Chloroquine Stimulated Tat Uptake

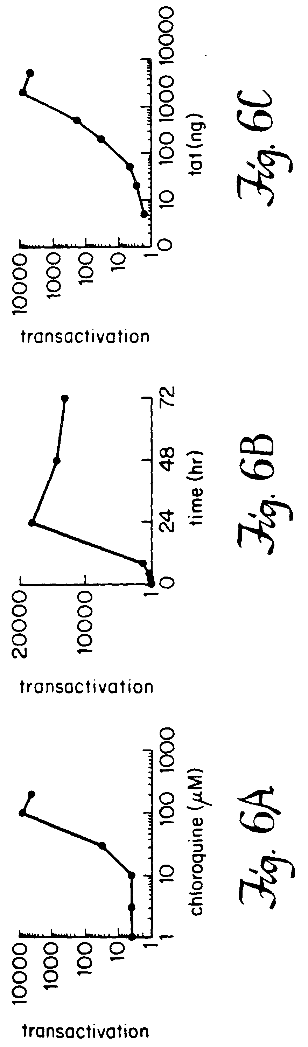

The parameters of chloroquine-stimulated Tat activity were studied in more detail. Figure 6A shows that the concentration dependence of chloroquine is a rather sharp dose response with maximum transactivation observed at 100 µM chloroquine. This concentration is typically used to raise vacuolar pH (Mellman, I. et al., Annu. Rev. Biochem. 55:663-700 (1986)).

The time course of Tat transactivation in the presence of chloroquine showed a plateau after 24 hours (Figure 6B), and transactivation in the presence of chloroquine increased with increasing Tat concentration (Figure 6C). Transactivation was detectable with Tat concentrations as low as 1 nM.

Controls were done to determine whether transactivation was dependent on an intact TAR site, to determine whether a heterologous promoter could be stimulated by Tat, and to determine whether any of the effects seen with chloroquine occurred when Tat was produced intracellularly. After transient transfection of HeLa cells with an HIV-LTR plasmid (p-167/+80; Rosen, C.A. et al., Cell 41:813-823 (1985)), high levels of transactivation were seen when Tat was introduced by cotransfection with a Tat expression plasmid (pSV2tat72), by scrape-loading purified Tat, or by treatment with Tat and chloroquine. However, expression from the HIV-LTR containing a mutant TAR site (p-167/+21; Rosen, C.A. et al.*,* Cell 41:813-823 (1985)) or from the SV40 early promoter (pSV2-CAT; Gorman, C.M. et al., Mol. Cell. Biol. 2:1044-1051 (1982)) was not stimulated when Tat was introduced by these methods. Thus, introducing Tat by scrape-loading or by uptake with chloroquine appears to transactivate the HIV-LTR by the same mechanism that occurs when Tat is produced intracellularly. Chloroquine had no effect when Tat was produced intracellularly; chloroquine treatment of HL3T1 cells transiently transfected with pSV2tat72 showed no additional transactivation.

### Example V RNA Isolation and Analysis

For the RNase protection experiment, total RNA was isolated by the hot acidic phenol method (Queen, C. and D. Baltimore, Cell 33:741-748 (1983)). HIV-1-specific probes for all hybridizations were prepared by in vitro transcription (with α-³²P-UTP) of an EcoRV-linearized plasmid containing the EcoRV (-120) to HindIII (+80) fragment of the viral LTR (cloned into the plasmid sp73; Promega). RNA probes were purified on Sephadex G-50 spin columns (Boehringer-Mannheim).

RNase protection experiments were performed as described (Ausubel, F.M. et al., Current Protocols in Molecular Biology (New York: John Wiley and Sons, 1987)). Twenty µg of cellular RNA were hybridized overnight with 5 X 10⁵ cpm of the RNA probe at 38°C in 40 µl of 80% formamide, 40 mM PIPES (pH 6.7), 200 mM NaCl, 1 mM EDTA. Single-stranded RNA was digested with RNase A (10 µg/ml) and RNase T1 (45 U/ml) (Boehringer-Mannheim) in 400 µl of 10 mM Tris-HCl (pH 7.5), 300 mM NaCl, 5 mM EDTA for 1 hour at room temperature. Protected fragments were analyzed by electrophoresis on 6% polyacrylamide-7M urea sequencing gels. Protected RNAs were visualized by autoradiography with intensifying screens and were quantitated using a Betascope 603 (Betagen).

### Example VI Localization of Tat by Fluorescence

Purified Tat protein was labeled at lysine residues with tetramethyl rhodamine isothiocyanate (TRITC) by incubating 200 µg of Tat (amino acids 1-72) in 0.1 M Na₂CO₃ pH 9.0 with 5 µg of TRITC dissolved in 5 µl dimethylsulfoxide (DMSO), for 8 hours at 4°C. Unreacted TRITC was quenched with 50 mM NH₄Cl. The pH was lowered to 7.0 with HCl and rhodamine-labeled Tat was purified from free TRITC by dialysis against 50 mM Tris, pH 7, 1 mM DTT.

HL3T1 cells were grown on glass coverslips and incubated for various lengths of time with rhodamine-conjugated Tat (TRITC-Tat) in DMEM. H938 cells in suspension were incubated with rhodamine-conjugated Tat in RPMI. Cells were washed three times with phosphate buffered saline and viewed live on a Zeiss Axiophot fluorescence microscope.

### Example VII Uptake of TatE2C Fusion Protein

Cell lines. The mouse embryo fibroblast cell line Balb/c 3T3 (clone A31; Aaronson and Todaro, J. Cell Physiol. 72:141-148 (1968)) was obtained from the American Type Culture Collection. HeLa cells were obtained from Dr. Alan Frankel (Whitehead Institute, MIT). Both cell lines were propagated in Dulbecco's minimal essential medium (GIBCO) supplemented with 10% donor calf serum (Hazelton) and 4 mM glutamine (Whittaker). Cells were grown in a 5.5% CO₂ incubator at 37°C. Passaging of cells was performed by washing with phosphate-buffered saline and treating with trypsin (both GIBCO) to remove cells from plates followed by addition of culture medium and dilution of cells into plates containing fresh culture medium.

The HeLa cell line containing a Tat responsive reporter construct (HeLa.318) was generated by the introduction and stable selection of plasmid pXB318 (described below) by electroporation as described by Chu et al. (Chu et al., Nucleic Acids Res. 15:1311-1326 (1987)). pXB318 DNA was electroporated together with the selectable marker pSV2-neo (Southern, E.M. and Berg, P. J. Mol. Appl. Genet. 1:327-341 (1982)). Stable transfectants were selected in the presence of G418 (Southern, E.M. and Berg, P. J. Mol. Appl. Genet. 1:327-341 (1982)), and the presence of pXB318 DNA was confirmed by Southern blot hybridization analysis (Southern, E.M., J. Mol. Biol. 98:503-517 (1975)).

Vector Constructions. All molecular cloning reactions were carried out by methods described by Maniatis et al. (Maniatis, T., Fritsch, E.F., and Sambrook, J., Molecular Cloning: A Laboratory Manual (Cold Spring Harbor Laboratory, NY (1982)), using enzymes obtained from New England Biolabs (Beverly, MA).

The TatE2C fusion protein (protein TatE2C), in which HIV Tat was fused to the carboxy terminal portion of BPV-1 E2, was expressed from the bacterial expression plasmid pFTE103. This plasmid was derived from ptat72 (see Example I) by insertion of a StyI-SpeI fragment which was isolated from vector pCO-E2 (Hawley-Nelson et al., EMBO J. 7:525-531 (1988)) and which encodes the C-terminal portion of the E2 protein. Four synthetic deoxyoligonucleotides were used in the construction described below in detail.

The plasmid ptat72 was cleaved with the restriction endonucleases NdeI and BamHI releasing the Tat encoding portion of the vector. The 4603 base pair (bp) vector fragment was purified by agarose gel electrophoresis, and a 169 base pair (bp) NdeI-AatII fragment of the Tat encoding fragment was isolated. The 3' portion of the E2C coding sequence was isolated as a 375 bp StyI-SpeI fragment from pCO-E2 (Hawley-Nelson et al., EMBO J. 7:525-531 (1988); obtained from Dr. Elliot Androphy, Tufts University/New England Medical Center Hospitals). The E2C fragment was connected to the Tat fragment and to the expression vector by use of two pairs of complementary deoxyoligonucleotides (synthesized according to standard procedures using an Applied Biosystems 380A DNA Synthesizer). One complementary pair of oligonucleotides was designed to join the AatII overhang of the Tat fragment to the StyI overhang of the E2C fragment: 
oligo 374-3
5′ CGTCCGCCGCAGGGATCCCAGACCCACCAGGTTCCGGTTACTCTGC 3′
3′ TGCAGCAGGCGGCGTCCCTAGGGTCTGGGTGGTCCAAGGCCAATGAGACGGTTC 5′
oligo 374-4
 A second pair of complementary oligonucleotides was designed to link the SpeI overhang of the E2C fragment to the BamHI overhang of the 4603 bp vector backbone isolated from ptat72: 
oligo 374-5
5′ CTAGTGGCTCGAGATTCCG 3′
3′ ACCGAGCTCTAAGGCCTAG 5′
oligo 374-6
 The Tat fragment, the E2C fragment and the two pairs of oligos were inserted into the 4603 bp ptat72 vector backbone to create pFTE103. The resulting fusion gene is designed to express a protein comprising amino acids 1 through 67 of Tat at the amino terminus followed by the C-terminal 105 amino acids of E2 (residues 306 through 410 of BPV01 E2).

The Tat responsive reporter construct pXB318 was constructed in three steps. The starting plasmid was pBG312 (Cate et al., Cell 45:685-698 (1986)). Two oligodeoxynucleotides were synthesized, which when annealed have an AatII-compatible overhang at the 5' end and an EcoRI-compatible overhang at the 3' end, and form a polylinker with internal XhoI, HindIII and BamHI restriction sites: 
5′ CTCGAGAAGCTTGACGGATCCG 3′
3′ TGCAGAGCTCTTCGAACTGCCTAGGCTTAA 5′
 pBG312 was cleaved with AatII and EcoRI to remove the promoter, and the above polylinker was inserted into the vector to form the promoterless vector pXB100. The HIV-1 long terminal repeat (LTR) from pU3R-III (Sodroski et al. Science 227:171-173 (1985)) was excised as a XhoI-HindIII fragment and was inserted into XhoI and HindIII sites of the polylinker of pXB100 to create pXB301. The human tissue plasminogen activator (tPA) cDNA reporter was excised as a BamHI fragment from pTPA114 (Fisher et al. J. Biol. Chem. 260:11223-11230 (1985)) and inserted into the BamHI site of pXB301 to create pXB318.

Expression and purification of TatE2C. The TatE2C fusion protein was expressed in E. coli using the vector pFTE103 and the T7 RNA polymerase expression system precisely as described by Studier et al. (Studier et al., Methods in Enzymology 185:60-89 (1990)).

Virtually all of the TatE2C protein was found in the insoluble fraction. The following purification was performed:
1. E. coli were pelleted, resuspended in ten packed cell volumes of 25 mM Tris-HCl pH 7.5, 1 mM EDTA, 10 mM DTT, and 1 mM PMSF and lysed with two passages through a French press.
2. The membrane fraction was pelleted by centrifugation at 10,000 rpm for 30 minutes.
3. This membrane fraction was resuspended in 6 M urea.
4. Solid guanidine-HCl was added to a final concentration of 6 M and DTT was added to a final concentration of 10 mM.
5. After 30 minutes at 37°C, the solution was clarified by centrifugation at 10,000 rpm for 30 minutes.
6. The sample was loaded onto an A.5 agarose gel filtration column in 6 M guanidine-HCl, 50 mM sodium phosphate pH 5.4, and 10 mM DTT.
7. TatE2C-containing fractions were loaded onto a C₁₈ reverse phase HPLC column and eluted with a gradient of 0-75% acetonitrile in 0.1% trifluoroacetic acid.

TatE2C protein appeared in a single peak. On protein gels, the TatE2C fusion protein migrated with an apparent molecular weight of 20,000 to 21,000 daltons.

Assay of TatE2C uptake by Tat activity. Uptake was detected either as Tat activity (activation of a Tat-dependent reporter in HeLa.318) or by indirect immunofluorescence using anti-E2 antibodies.

Tat activity was determined by adding Tat protein (amino acids 1-72) or TatE2C fusion protein at 2.5-250 nM along with chloroquine at 0.1 mM to the culture medium of HeLa.318 cells in 24 well plates essentially by the method of Frankel and Pabo (Frankel, A.D. and Pabo, C.O., Cell 55:1189-1193 (1988)). The culture medium was harvested 24 hours later and assayed for tPA activity by the method of Granelli-Piperno and Reich (J. Exp. Med. 148:223-234 (1978)). Cell numbers were determined and tPA secretion was expressed as ng/10⁶ cells per day. tPA secretion was undetectable in the absence of added Tat or TatE2C protein (less than 0.1 ng/10⁶ cells per day).

Assay of TatE2C uptake by E2-specific immunofluorescence. For indirect immunofluorescence, mouse 3T3 cells were seeded into LAB-TEK four chamber tissue culture chamber/slides. On the next day, TatE2C protein and chloroquine were added to the culture medium to final concentrations of 250 nM and 0.1 mM, respectively. Six hours later, immunofluorescence was performed as follows:
1. Medium was removed and wells were washed twice with phosphate-buffered saline (PBS).
2. Cells were fixed by treatment with 3.5% formaldehyde for 10 minutes at room temperature.
3. Cells were permeabilized in 0.2% Triton X-100/2% bovine serum albumin (BSA) in PBS with 1 mM MgCl₂/0.1 mM CaCl₂ (PBS+) for 5 minutes at room temperature.
4. Cells were blocked by treatment with whole goat serum (Cappel #5506-1380) at a 1:30 dilution in PBS+/2% BSA for one hour at 4°C.
5. The primary antibody was an affinity purified rabbit polyclonal which had been raised by injection of purified protein E2.C85 (in this case the carboxy terminal 85 amino acids expressed in bacteria using the T7 polymerase expression system) into a rabbit, followed by purification by passage of the bleed over an E2 affinity column. This antibody was added to the wells at a 1:100 dilution in PBS+/2% BSA for one hour at 4°C.
6. The secondary antibody was a rhodamine conjugated goat anti-rabbit IgG (Cappel #2212-0081). This antibody was added at a 1:100 dilution in PBS+/0.2% BSA for 30 minutes at 4°C.
7. Wells were washed three times with PBS+/0.2% Tween-20/2% BSA.
8. Slides were mounted in 50% glycerol in PBS and viewed with a fluorescent microscope with a rhodamine filter.

As a control, purified E2C protein (the carboxy terminal 85 amino acids which were found to be recognized by the polyclonal antibody preparation) was added to wells in the same manner as the TatE2C fusion protein.

## Claims

1. A process for delivering a non-tat molecule of interest into a eukaryotic cell, comprising the step of contacting a non-tat molecule of interest-tat protein conjugate with the extracellular surface of a cell so that the conjugate enters into the cell.

2. A process for delivering a non-tat molecule of interest into a eukaryotic cell nucleus, comprising the step of contacting a non-tat molecule of interest-tat protein conjugate with the extracellular surface of a cell so that the conjugate enters into the cell and passes into the nucleus.

3. The process according to claim 1 or 2, wherein the tat protein is selected from the group consisting of amino acids 1-67, amino acids 1-72 and amino acids 1-58 of the HIV tat protein, as set forth in Figure 1.

4. The process according to claim 1 or 2, wherein the molecule of interest is selected from the group consisting of proteins, peptides and nucleic acids.

5. The process according to claim 4, wherein the molecule of interest is a protein selected from the group consisting of regulatory factors, antibodies, enzymes and toxins.

6. The process according to claim 1 or 2, wherein step (b) further comprises the step of contacting the target cell with a stabilizing agent to inhibit proteolysis of the molecule of interest-tat protein conjugate.

7. The process according to claim 6, wherein the stabilizing agent is selected from the group consisting of a metal and a lysosomotrophic agent.

8. The process according to claim 7, wherein the lysosomotrophic agent is selected from the group consisting of chloroquine, amantadine, monensin, methylamine and ammonium chloride.

9. The process according to claim 1 or 2, wherein step (b) further comprises contacting the cell with a basic peptide.

10. The process according to claim 9, wherein the basic peptide is selected from the group consisting of protamine and amino acids 38-58 of the HIV tat protein.

11. The process according to claim 1 or 2, wherein the molecule of interest is a drug.

12. The process according to claim 11, wherein the drug is a peptide analog or small molecule enzyme inhibitor.

13. The process according to claim 1 or 2, wherein the molecule of interest is a diagnostic probe.

14. The use of a molecule of interest-tat protein conjugate comprising a non-tat molecule of interest covalently linked to a tat protein or peptide having cellular uptake activity for the preparation of a pharmaceutical composition to be used to deliver the non-tat molecule of interest into a cell.

15. The use of a conjugate according to claim 14, wherein the tat protein is selected from the group consisting of amino acids 1-67, amino acids 1-72 and amino acids 1-58 of the HIV tat protein, as set forth in Figure 1.

16. The use of a conjugate according to claim 14 or 15, wherein the molecule of interest is selected from the group consisting of proteins, peptides, and nucleic acids.

17. The use of a conjugate according to claim 16, wherein the molecule of interest is a polypeptide selected from the group consisting of regulatory factors, antibodies, enzymes and toxins.

18. The use of a conjugate according to claim 14 or 15, wherein the molecule of interest is a drug.

19. The use of a conjugate according to claim 18, wherein the drug is a peptide analog or small molecule enzyme inhibitor.

20. The use of a conjugate according to claim 14 or 15, wherein the molecule of interest is a diagnostic probe.

21. The use of a conjugate according to any one of claims 14-20, wherein the pharmaceutical composition further comprises a stabilizing agent.

22. The use of a conjugate according to claim 21, wherein the stabilizing agent is selected from the group consisting of a metal and a lysosomotrophic agent.

23. The use of a conjugate according to claim 22, wherein the lysosomotrophic agent is selected from the group consisting of chloroquine, amantadine, monensin, methylamine and ammonium chloride.

24. The use of a conjugate according to claim 21, wherein the pharmaceutical composition further comprises a basic peptide.

25. The use of a conjugate according to claim 24, wherein the basic peptide is selected from the group consisting of protamine and amino acids 38-58 of the HIV tat protein.

## Patentansprüche

1. Verfahren zur Einschleusung eines gewünschten nicht-tat-Moleküls in eine eukaryotische Zelle, umfassend den Schritt des Inkontaktbringens eines Konjugats aus gewünschtem nicht-tat-Molekül/tat-Protein mit der extrazellulären Oberfläche einer Zelle, so daß das Konjugat in die Zelle eindringt.

2. Verfahren zur Einschleusung eines gewünschten nicht-tat-Moleküls in einen eukaryotischen Zellkern, umfassend den Schritt des Inkontaktbringens eines Konjugats aus einem gewünschten nicht-tat-Molekül/tat-Protein mit der extrazellulären Oberfläche einer Zelle, so daß das Konjugat in die Zelle eindringt und in den Kern gelangt.

3. Verfahren nach Anspruch 1 oder 2, wobei das tat-Protein ausgewählt ist aus den Aminosäuren 1-67, 1-72 oder 1-58 des HIV-tat-Proteins wie in Figur 1 gezeigt.

4. Verfahren nach Anspruch 1 oder 2, wobei das gewünschte Molekül ausgewählt ist aus Proteinen, Peptiden oder Nucleinsäuren.

5. Verfahren nach Anspruch 4, wobei das gewünschte Molekül ein Protein ist, ausgewählt aus regulatorischen Faktoren, Antikörpern, Enzymen oder Toxinen.

6. Verfahren nach Anspruch 1 oder 2, wobei Schritt (b) außerdem den Schritt des Inkontaktbringens der Zielzelle mit einem Stabilisierungsmittel zur Hemmung der Proteolyse des Konjugats aus dem gewünschten Molekül/tat-Protein umfaßt.

7. Verfahren nach Anspruch 6, wobei das Stabilisierungsmittel ausgewählt ist aus einem Metall oder einem lysosomotrophen Mittel.

8. Verfahren nach Anspruch 7, wobei das lysosomotrophe Mittel ausgewählt ist aus Chloroquin, Amantadin, Monensin, Methylamin oder Ammoniumchlorid.

9. Verfahren nach Anspruch 1 oder 2, wobei Schritt (b) außerdem das Inkontaktbringen der Zelle mit einem basischen Peptid umfaßt.

10. Verfahren nach Anspruch 9, wobei das basische Peptid ausgewählt ist aus Protamin oder den Aminosäuren 38-58 des HIV-tat-Proteins.

11. Verfahren nach Anspruch 1 oder 2, wobei das gewünschte Molekül ein Arzneistoff ist.

12. Verfahren nach Anspruch 11, wobei der Arzneistoff ein Peptidanalogon oder ein enzymhemmendes kleines Molekül ist.

13. Verfahren nach Anspruch 1 oder 2, wobei das gewünschte Molekül eine diagnostische Sonde ist.

14. Verwendung eines Konjugats aus einem gewünschten Molekül/tat-Protein, umfassend ein gewünschtes nicht-tat-Molekül, das mit einem tat-Protein oder -Peptid mit einer Aktivität für zelluläre Aufnahme kovalent verknüpft ist, zur Herstellung eines Arzneimittels zur Verwendung für die Einschleusung des gewünschten nicht-tat-Moleküls in eine Zelle.

15. Verwendung eines Konjugats nach Anspruch 14, wobei das tat-Protein ausgewählt ist aus den Aminosäuren 1-67, 1-72 oder 1-58 des HIV-tat-Proteins wie in Figur 1 gezeigt.

16. Verwendung eines Konjugats nach Anspruch 14 oder 15, wobei das gewünschte Molekül ausgewählt ist aus Proteinen, Peptiden oder Nucleinsäuren.

17. Verwendung eines Konjugats nach Anspruch 16, wobei das gewünschte Molekül ein Polypeptid ist, ausgewählt aus regulatorischen Faktoren, Antikörpern, Enzymen oder Toxinen.

18. Verwendung eines Konjugats nach Anspruch 14 oder 15, wobei das gewünschte Molekül ein Arzneistoff ist.

19. Verwendung eines Konjugats nach Anspruch 18, wobei der Arzneistoff ein Peptidanalogon oder ein enzymhemmendes kleines Molekül ist.

20. Verwendung eines Konjugats nach Anspruch 14 oder 15, wobei das gewünschte Molekül eine diagnostische Sonde ist.

21. Verwendung eines Konjugats nach einem der Ansprüche 14 bis 20, wobei das Arzneimittel außerdem ein Stabilisierungsmittel umfaßt.

22. Verwendung eines Konjugats nach Anspruch 21, wobei das Stabilisierungsmittel ausgewählt ist aus einem Metall oder einem lysosomotrophen Mittel.

23. Verwendung eines Konjugats nach Anspruch 22, wobei das lysosomotrophe Mittel ausgewählt ist aus Chloroquin, Amantadin, Monensin, Methylamin oder Ammoniumchlorid.

24. Verwendung eines Konjugats nach Anspruch 21, wobei das Arzneimittel außerdem ein basisches Peptid umfaßt.

25. Verwendung eines Konjugats nach Anspruch 24, wobei das basische Peptid ausgewählt ist aus Protamin oder den Aminosäuren 38-58 des HIV-tat-Proteins.

## Revendications

1. Procédé de délivrance d'une molécule d'intérêt non-tat à une cellule eucaryote, qui comprend l'étape de mise en contact d'un molécule d'intérêt non-tat d'un conjugué de protéine tat, avec la surface extracellulaire d'une cellule de façon que le conjugué pénètre dans la cellule.

2. Procédé de délivrance d'une molécule d'intérêt non-tat dans un noyau de cellule eucaryote qui comprend l'étape de mise en contact de la molécule d'intérêt non-tat d'un conjugué de protéine tat avec la surface extracellulaire d'une cellule de façon que le conjugué pénètre dans la cellule et passe dans le noyau.

3. Procédé selon la revendication 1 ou 2,
caractérisé en ce que,
la protéine tat est choisie dans le groupe qui consiste en les aminoacides 1-67, les aminoacides 1-72 et les aminoacides 1-58 de la protéine tat de HIV comme établi dans la figure 1.

4. Procédé selon la revendication 1 ou 2,
caractérisé en ce que,
la molécule d'intérêt est choisie dans le groupe consistant en des protéines, des peptides et des acides nucléiques.

5. Procédé selon la revendication 4,
caractérisé en ce que,
la molécule d'intérêt est une protéine choisie dans le groupe consistant en des facteurs de régulation, des anticorps, des enzymes et des toxines.

6. Procédé selon la revendication 1 ou 2,
caractérisé en ce que,
l'étape b) comprend en outre l'étape de mise en contact de la cellule cible avec un agent stabilisant pour inhiber la protéolyse de la molécule d'intérêt d'un conjugué de protéine tat.

7. Procédé selon la revendication 6,
caractérisé en ce que,
l'agent stabilisant est choisi dans le groupe consistant en un métal et un agent lysosomotrophique.

8. Procédé selon la revendication 7,
caractérisé en ce que,
l'agent lysosomotrophique est choisi dans le groupe consistant en la chloroquine, l'amantadine, la monensine, la méthylamine et le chlorure d'ammonium.

9. Procédé selon la revendication 1 ou 2,
caractérisé en ce que,
l'étape b) comprend en outre la mise en contact de la cellule avec un peptide basique.

10. Procédé selon la revendication 9,
caractérisé en ce que,
le peptide basique est choisi dans le groupe consistant en la protamine et les aminoacides 38-58 de la protéine tat de HIV.

11. Procédé selon la revendication 1 ou 2,
caractérisé en ce que
la molécule d'intérêt est un médicament.

12. Procédé selon la revendication 11,
caractérisé en ce que
le médicament est un analogue de peptide ou un inhibiteur d'enzyme à petite molécule.

13. Procédé selon la revendication 1 ou 2,
caractérisé en ce que
la molécule d'intérêt est une sonde de diagnostic.

14. Utilisation d'une molécule d'intérêt d'un conjugué de protéine tat qui comprend une molécule d'intérêt non-tat, liée d'une manière covalente à une protéine tat ou à un peptide ayant une activité de capture cellulaire, pour la préparation d'une composition pharmaceutique qui doit être utilisée pour délivrer la molécule d'intérêt non-tat dans une cellule.

15. Utilisation d'un conjugué selon la revendication 14,
caractérisée en ce que,
la protéine tat est choisie dans le groupe consistant en des aminoacides 1-67, des aminoacides 1-72 et des aminoacides 1-58 de la protéine tat de HIV, comme établi dans la figure 1.

16. Utilisation d'un conjugué selon la revendication 14 ou 15,
caractérisée en ce que,
la molécule d'intérêt est choisie dans le groupe consistant en des protéines, des peptides et des acides nucléiques.

17. Utilisation d'un conjugué selon la revendication 16,
caractérisée en ce que,
la molécule d'intérêt est un polypeptide choisi dans le groupe consistant en des facteurs de régulation, des anticorps, des enzymes et des toxines.

18. Utilisation d'un conjugué selon la revendication 14 ou 15,
caractérisée en ce que,
la molécule d'intérêt est un médicament.

19. Utilisation d'un conjugué selon la revendication 18,
caractérisée en ce que,
le médicament est un analogue de peptide ou un inhibiteur d'enzyme à petite molécule.

20. Utilisation d'un conjugué selon la revendication 14 ou 15,
caractérisée en ce que la molécule d'intérêt est une sonde de diagnostic.

21. Utilisation d'un conjugué selon l'une quelconque des revendications 14 à 20,
caractérisée en ce que,
la composition pharmaceutique comprend en outre un agent stabilisant.

22. Utilisation d'un conjugué selon la revendication 21,
caractérisée en ce que,
l'agent stabilisant est choisi dans le groupe consistant en un métal et un agent lysosomotrophique.

23. Utilisation d'un conjugué selon la revendication 22,
caractérisée en ce que,
l'agent lysosomotrophique est choisi dans le groupe consistant en la chloroquine, l'amantadine, la monensine, la méthylamine et le chlorure d'ammonium.

24. Utilisation d'un conjugué selon la revendication 21,
caractérisée en ce que,
la composition pharmaceutique comprend en outre un peptide basique.

25. Utilisation d'un conjugué selon la revendication 24,
caractérisée en ce que,
le peptide basique est choisi dans le groupe qui consiste en la protamine et les aminoacides 38-58 de la protéine tat de HIV.
